(19) Europäisches Patentamt European Patent Office Office européen des brevets

(11) **EP 1 784 422 B1**

(12) **EUROPÄISCHE PATENTSCHRIFT**

(45) Veröffentlichungstag und Bekanntmachung des Hinweises auf die Patenterteilung:
**27.10.2010 Patentblatt 2010/43**

(21) Anmeldenummer: **05778889.5**

(22) Anmeldetag: **26.08.2005**

(51) Int Cl.:
***C07K 14/605*** *(2006.01)*

(86) Internationale Anmeldenummer:
**PCT/DE2005/001503**

(87) Internationale Veröffentlichungsnummer:
**WO 2006/024275 (09.03.2006 Gazette 2006/10)**

(54) **ERFINDUNG BETREFFEND GLP-1 UND EXENDIN**

GLP-1 AND EXENDIN RELATED INVENTION

INVENTION CONCERNANT LE GLP-1 ET L'EXENDINE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **03.09.2004 DE 102004043153**

(43) Veröffentlichungstag der Anmeldung:
**16.05.2007 Patentblatt 2007/20**

(60) Teilanmeldung:
**08020703.8 / 2 070 946**

(73) Patentinhaber: **Philipps-Universität Marburg**
**35032 Marburg (DE)**

(72) Erfinder:
• **GOTTHARDT, Martin**
**35274 Kirchhain (DE)**
• **BEHE , Martin**
**35043 Marburg (DE)**
• **BEHR, Thomas**
**35043 Marburg-Cappel (DE)**
• **GÖKE, Burkhard, J.**
**82131 Gauting (DE)**

(74) Vertreter: **Buchhold, Jürgen**
**Patentanwälte Olbricht & Buchhold**
**Am Weinberg 15**
**35096 Weimar/Lahn (DE)**

(56) Entgegenhaltungen:
WO-A-91/01144          US-A1- 2003 073 626
US-A1- 2003 232 761

• XIAO Q ET AL: "Biological activities of glucagon-like peptide-1 analogues in vitro and in vivo" BIOCHEMISTRY, AMERICAN CHEMICAL SOCIETY. EASTON, PA, US, vol. 40, no. 9, 6 March 2001 (2001-03-06), pages 2860-2869, XP002277645 ISSN: 0006-2960
• KNUDSEN LOTTE B ET AL: "Potent derivatives of glucagon-like peptide-1 with pharmacokinetic properties suitable for once daily administration" JOURNAL OF MEDICINAL CHEMISTRY, AMERICAN CHEMICAL SOCIETY. WASHINGTON, US, vol. 43, no. 9, 4 May 2000 (2000-05-04), pages 1664-1669, XP002201014 ISSN: 0022-2623
• DEACON C F ET AL: "Dipeptidyl peptidase IV resistant analogues of glucagon-like peptide-1 which have extended metabolic stability and improved biological activity." DIABETOLOGIA. MAR 1998, vol. 41, no. 3, March 1998 (1998-03), pages 271-278, XP002202152 ISSN: 0012-186X
• GÖKE R ET AL: "Solubilization of active GLP-1 (7-36)amide receptors from RINm5F plasma membranes." FEBS LETTERS. 6 APR 1992, vol. 300, no. 3, 6 April 1992 (1992-04-06), pages 232-236, XP002374756 ISSN: 0014-5793
• ABELLO J ET AL: "Stimulation of glucagon-like peptide-1 secretion by muscarinic agonist in a murine intestinal endocrine cell line." ENDOCRINOLOGY. MAY 1994, vol. 134, no. 5, May 1994 (1994-05), pages 2011-2017, XP009064445 ISSN: 0013-7227

EP 1 784 422 B1

- CHAHRZAD MONTROSE-RAFIZADEH ET AL: "High Potency Antagonists of the Pancreatic Glucagon-like Peptide-1 Receptor" JOURNAL OF BIOLOGICAL CHEMISTRY, AMERICAN SOCIETY OF BIOLOCHEMICAL BIOLOGISTS, BIRMINGHAM,, US, vol. 272, no. 34, 22 August 1997 (1997-08-22), pages 21201-21206, XP002247450 ISSN: 0021-9258
- BEHR T M ET AL: "IMAGING TUMORS WITH PEPTIDE-BASED RADIOLIGANDS" QUARTERLY JOURNAL OF NUCLEAR MEDICINE, MILAN, IT, vol. 45, no. 2, 2001, pages 189-200, XP008023979 ISSN: 1125-0135
- STENNICKE H R ET AL: "C-terminal incorporation of fluorogenic and affinity labels using wild-type and mutagenized carboxypeptidase Y" ANALYTICAL BIOCHEMISTRY, ACADEMIC PRESS, SAN DIEGO, CA, US, vol. 248, no. 1, 15 May 1997 (1997-05-15), pages 141-148, XP002298918 ISSN: 0003-2697

**EP 1 784 422 B1**

**Beschreibung**

Bei der Lokalisationsdiagnostik von gastroenteropankreatischen neuroendokrinen Tumoren wird als wichtigste diagnostische Methode neben dem Ultraschall die Somatostatin Rezeptor Szintigraphie (SRS) eingesetzt. Das Prinzip ist hier die spezifische Darstellung von Tumoren mit Hilfe von radioaktiv markierten Peptiden, die in die Tumorzellen aufgenommen werden. Sodann kann mit Hilfe von Gammakameras die Anreicherung der Radioaktivität im Tumorgewebe bildlich nachgewiesen werden. Wenn ein Tumortyp einen für die SRS erforderlichen Rezeptor z.B. für das Somatostatin-Analogon Octreotide® besitzt, ist ein Nachweis dieser Tumoren problemlos möglich. Werden entsprechende Rezeptoren jedoch nicht exprimiert, entziehen sie sich dem szintigraphischen Nachweis. Neben der Lokalisationsdiagnostik ermöglichen radioakiv markierte Peptide aber auch einen Ansatz zur Tumorbehandlung, indem man durch Markierung von Somatostatin-Analoga z.B. Octreotide® mit einem geeigneten Radionuklid (α- oder β-Strahler) eine spezifische rezeptorgerichtete Radiopeptidtherapie durchführen kann. Da die entsprechenden Radionuklide chemisch so vom Peptid gebunden werden (z.B. über Komplexierung mit einem zuvor an das Peptid gebundenen Metallchelator), dass sie zwar in die Tumorzelle aufgenommen aber nicht mehr ausgeschleust werden können, ergibt sich eine hohe spezifische Anreicherung im Tumorgewebe.

Allerdings exprimieren eine ganze Reihe von neuroendokrinen Tumoren (NET) darunter Insulinome und kleinzellige Bronchialkarzinome nicht die erforderlichen Subtypen des Somatostatinrezeptors, die für die SRS oder die Radiopeptidetheraphie mit dem Somatostatin-Analogon Octreotide® notwendig sind. Insbesondere Insulinome entziehen sich in einem erheblichen Prozentsatz der szintigraphischen Diagnostik. Auch bei kleinzelligen Bronchialkarzinomen stellt die SRS kein geeignetes Verfahren dar, da zwar die Primärtumore häufig darstellbar sind, die Metastasen jedoch nicht, da sie die Rezeptorexpression verloren haben. Somit sind sie auch keiner Radiopeptidtherapie zugänglich, die ein interessantes zusätzliches oder alternatives Therapieverfahren darstellt. Daher besteht der Bedarf an einem geeigneten Peptid, das von den genannten Tumoren aufgenommen wird.

Das Inkretinhormon Glucagon-Like Peptid-1 (GLP-1) sowie seine Analoga Exendin 3 und Exendin 4 (aus dem Speichel des Gilamonsters (Heloderma horridum und Heloderma suspectum)) sind Peptide, für die Insulinome und kleinzellige Bronchialkarzinome - neben vielen anderen Tumorarten - Rezeptoren exprimieren. Insulinome stammen von den insulinproduzierenden β-Zellen in den Langerhans'schen Inseln des Pankreas ab, in denen GLP-1 sowie Exendin 3 und Exendin 4 eine postprandiale Insulinsekretion auslösen.

**Stand der Technik**

Zur Verwendung von Glucagon-Like Peptide-1 (GLP-1) in der Szintigraphie ist eine Markierung der Peptide erforderlich. Die Verfahren dazu und zur Markierung von Proteinen mit Radionukliden sind dem Fachmann bekannt und in zahlreichen Patentschriften (z.B. DE 690 18 226 T2) und wissenschaftlichen Arbeiten belegt. Die dabei beschriebenen Peptide zur Anwendung in der diagnostischen Bildgebung und zur Vermittlung von therapeutisch wirksamen Molekülen in pathologisches Gewebe werden in der Regel am N-terminalen Ende über ein Amin in das Peptid eingeführt. Die Peptide müssen dabei bezüglich der Stabilisierung weiter modifiziert werden.

Das in der US 2003/0232761A1 verwendete GLP-1 und sein Derivat GLP-1(7-37) sind beispielsweise am N-terminalen Ende über ein Amin modifiziert. Somit steht das N-terminale Ende von GLP-1 für die Bindung an einen GLP-1-Rezeptor nicht mehr zur Verfügung, so dass mit diesen Peptiden nur eine ungenügende Rezeptorbindung und Intemalisierung möglich ist, diese Peptide also nicht zur Verwendung in der Radiopeptidtherapie von Insulinomen und kleinzelligen Bronchialkarzinomen geeignet sind. Eine Mutation z.B. ein Austausch einer Aminosäure innerhalb der Peptidsequenz von GLP-1 und Exendin-3 oder Exendin-4 und deren mögliche Modifikation durch ein Therapeutikum oder ein signalgebendes Molekül führt erfahrungsgemäß meist dazu, dass die Struktur des Peptides so gestört wird, dass keine Bindung an den Rezeptor mehr möglich ist.

Eine weitere Methode zur Modifikation von GLP-1, ohne Beeinflussung des N-Terminus ist derzeit nicht bekannt. Darüber hinaus sind keine GLP-1 Derivate bekannt, die markiert oder unmarkiert zum Einsatz in der Radiotherapie von Insulinomen und kleinzelligen Bronchialkarzinomen geeignet sind.

**Aufgabe**

Aufgabe der vorliegenden Erfindung ist daher, den Mangel im Stand der Technik zu beseitigen und Peptide bereitzustellen, die markiert werden können und auch mit dieser Markierung an den GLP-1-Rezeptor binden und die zur Herstellung eines Mittels für Diagnostik und Therapie von Erkrankungen, bei denen die GLP-1 Rezeptorexpression eine Rolle spielt, eingesetzt werden.

3

**Lösung der Aufgabe**

Diese Aufgabe wird erfindungsgemäß durch, durch Peptidderivate von GLP-1, welche ganz oder teilweise die Aminosäuresequenz

$(H)-_1His-_2Asp-_3Glu-_4Phe-_5Glu-_6Arg-_7His-_8Ala-_9Glu-_{10}Gly-_{11}Thr-_{12}Phe-_{13}Thr-_{14}Ser-_{15}Asp-_{16}Val-_{17}Ser-_{18}Ser-_{19}Tyr-_{20}Leu-_{21}Glu-_{22}Gly-_{23}Gln-_{24}Ala-_{25}Ala-_{26}Lys-_{27}Glu-_{28}Phe-_{29}Ile-_{30}Ala-_{31}Trp-_{32}Leu-_{33}Val-_{34}Lys-_{35}Gly-_{36}Arg-_{37}Gly-(OH)$

des Peptides GLP-1(1-37) beinhalten gelöst, die gemäß der Erfindung am C-Terminus über ein Amin modifiziert sind und über den N-Terminus an den GLP-1-Rezeptor binden und die aufgebaut sind aus GLP-1(x-y)A wobei x die Zahl 7 annimmt und wobei y de Zahl 36 ist, und wobei A eine am C-Terminus lokalisierte Attachementgruppe zur Bindung von Signalmolekülen ist und wobei die Attachementgruppe A Lysin ist. Diese Peptidderivate werden unmarkiert oder markiert zur Herstellung eines Mittels zur Diagnostik und Therapie gut- und bösartiger Erkrankungen, bei denen die GLP-1 Rezeptorexpression eine Rolle spielt, eingesetzt.

Mit Hilfe dieser Peptidderivate von GLP-1, wird die Herstellung eines Mittels zur szintigraphischen Anwendung realisiert, das zur Diagnostik und Therapie von GLP-1-Rezeptor exprimierenden Tumoren, darunter NET (insbesondere von Insulinomen) und kleinzelligen Bronchialkarzinomen eingesetzt wird.

Dies ist erstmals möglich, da die erfindungsgemäßen Peptidderivate über ein Amin am C-Terminus modifiziert sind, und so der N-Terminus für die Bindung an den GLP-1-Rezeptor zur Verfügung steht.

Durch die Bindung der erfindungsgemäßen z.B. radioaktiv markierten Peptidderivate von GLP-1, an den GLP-1-Rezeptor ist die Darstellung von GLP-1-Rezeptor exprimierenden Tumoren möglich und damit erfolgt eine erhebliche Verbesserung der Patientenversorgung. In erster Linie sind gastroenteropankreatische NET, wie Insulinome, bei denen zur Zeit kein nicht-invasives Verfahren mit einer ausreichenden Sensitivität verfügbar ist oder im Bereich der Lunge lokalisierte kleinzellige Bronchialkarzinome, bei denen die spezifische Differenzierung zwischen entzündlichen Prozessen und Tumoren bzw. Metastasen ebenfalls derzeit durch kein nicht-invasives Verfahren möglich ist.

Weiterhin wird mittels der erfindungsgemäßen Peptidderivate die Dichte Insulin-produzierender Zellen im Pankreas dargestellt sowie die Expression von GLP-1-Rezeptoren *in vivo* und *in vitro.* Dies ist z.B. bei der Darstellung GLP-1-Rezeptoren exprimierender Zellen beim Diabestes mellitus eine *in vivo*-Darstellung, da dies die Zellen sind, die auch Insulin sezemieren. Die Darstellung der GLP-1-Rezeptordichte im Pankreas ist besonders bei Patienten mit Diabetes mellitus während und nach der Therapie mit Medikamenten wichtig.

Zusätzlich wird die Verteilung von GLP-1-Rezeptoren in malignen und benignen Geweben dargestellt. Fragestellungen sind hier sowohl klinischer als auch wissenschaftlicher Natur, da es noch keine umfassenden *in-vivo* Daten zur GLP-1-Rezeptorverteilung beim Menschen gibt.

Die Erfindung hat also den Vorteil, dass Peptidderivate von GLP-1 (Glucagon-like Peptide-1) zur Herstellung eines Mittels insbesondere zur rezeptorgerichteten spezifischen Darstellung und Therapie insbesondere von NET, hier besonders von Insulinomen und kleinzelligen Bronchialkarzinomen eingesetzt werden.

Insbesondere bei der Diagnostik von kleinzelligen Bronchialkarzinomen ist eine GLP-1-Rezeptorszintigraphie anwendbar und erlaubt erstmals die spezifische Detektion von metastatisch befallenen Lymphknoten (entzündlich veränderte Lymphknoten versus befallene Lymphknoten).

Die Verwendung der erfindungsgemäßen Peptidderivate von GLP-1 (Glucagon-like Peptide-1) erfolgt weiterhin als Mittel zur Diagnostik und Therapie aller gut- und bösartiger Erkrankungen, bei denen die GLP-1 Rezeptorexpression eine Rolle spielt, im Besonderen als Kontrastmittel mit der Magnetresonanztomographie (MRT), als radioaktives Mittel im der Szintigraphie (SPECT, Single Photonen Computer Tomographie) bzw. Radiopetidtheraphie, in der PET (Positronen Emissions Tomographie), rezeptorvermittelten Chemotherapie und der optischen Diagnostik. Wobei unter optischer Diagnostik die Anregung eines fluoreszierenden Moleküls durch eine bestimmte Wellenlänge, welche eine nachfolgende Emission von Licht einer anderen Wellenlänge auslöst, verstanden wird. Dabei wird die emittierte Wellenlänge detektiert.

Es ist dem Fachmann leicht möglich, die Art der Markierung am C-terminalen Terminus der Peptidderivate von GLP-1 (Glucagon-like Peptide-1) in Abhängigkeit von der gewünschten Anwendung auszuwählen, die z.B. für die Szintigraphie bzw. Radiotherapie aus radioaktiven Nukliden, für das Kontrastmittel in der Magnetresonanztomographie (MRT) beispielsweise aus Gadolinum, für endoskopische oder wissenschaftliche Untersuchungen aus Fluoreszenzfarbstoffen besteht.

Erfindungsgemäß werden unter malignen Erkrankungen bösartige Erkrankungen verstanden, bei denen die betroffenen Gewebe Veränderungen ihres Differenzierungsgrades gegenüber gesunden Geweben aufweisen, invasives Wachstum zeigen oder deren Gewebe sich über Blut- oder Lymphbahnen ausbreitet. Hierzu gehören alle neuroendokrinen Tumoren, insbesondere die des Gastrointestinaltraktes; insbesondere auch Insulinome, Bronchialkarzinome, Pankreaskarzinom und alle anderen bösartigen Erkrankungen, die mit einer Überexpression des GLP-1-Rezeptors verbunden sind.

Erfindungsgemäß werden unter benignen Erkrankungen gutartige Erkrankungen verstanden, die sich dadurch auszeichnen, dass die betroffenen Gewebe ihren Differenzierungsgrad nicht wesentlich verlieren, kein invasives Wachstum zeigen und keine Gewebeabsiedlungen über Lymph- oder Blutstrom bilden. Hierzu gehört z.B. Diabetes mellitus aber auch Störungen des Essverhaltens oder der Psyche.

**Charakterisierung der Peptidderivate und chimären Peptide**

Überraschenderweise wurde gefunden, dass Peptidderivate von GLP-1, die am C-Terminus über Amin modifiziert sind, über einen N-Terminus an den GLP-1-Rezeptor binden. Sie zeigen sogar eine dem natürlichen Peptid ähnliche sehr hohe Affinität zum GLP-1-Rezeptor. Experimente mit tumortragenden Nacktmäusen zeigen eine spezifische Aufnahme in GLP-1-Rezeptor-positives Tumorgewebe.

Die erfindungsgemäßen Peptidderivate sowie die chimären Peptide werden unmarkiert oder über einen Chelator am C-terminalen Amin markiert als Mittel zur Diagnostik und Therapie gut- und bösartiger Erkrankungen, bei denen die GLP-1 Rezeptorexpression eine Rolle spielt, eingesetzt. Die Art der Markierung besteht dabei insbesondere aus einem Radiometall, einem MRT-Kontrastmittel, einem fluoreszierenden Chromophor oder einem Chemotherapeutikum.

Der Vorgang und die Verfahren zur Markierung sind dem Fachmann geläufig (z.B. DE 690 18 226 T2) und erfolgen beispielsweise durch Kopplung von Radionukliden, amagnetischen Metallen und andere MRT-Kontrastmitteln oder Fluoreszenzfarbstoffen, so dass die Rezeptorbindung oder Internalisierung der erfindungsgemäßen Peptidderivate sowie der chimären Pepide nicht beeinträchtigt wird und der GLP-1-rezeptorbindende N-Terminus ungebunden bleibt.

Die Aminosäuresequenzen des Ausgangspeptides:

**GLP-1:**

H-His-Asp-Glu-Phe-Glu-Arg-His-Ala-Glu-Gly-Thr-Phe-Thr-Ser-Asp-
1　2　3　4　　5　6　7　8　9　10　11　12　13　14　15

Val-Ser-Ser-Tyr-Leu-Glu-Gly-Gln-Ala-Ala-Lys-Glu-Phe-Ile-Ala-Trp-
16　17　18　19　20　21　22　23　24　25　26　27　28　29　30　31

Leu-Val-Lys-Gly-Arg-Gly-OH
32　33　34　35　36　37

Erfindungsgemäß werden folgende Peptidderivate von GLP-1 (1-37) hergestellt:

**GLP-1 (x-y)A$^{37}$**

Wobei gilt:

x = Aminosäure 7 der GLP-1 Aminosäuresequenz

y = Aminosäure 36 der GLP-1 Aminosäuresequenz

A = Attachementgruppe bestehend aus einer oder mehreren Aminosäuren bzw. deren Derivate als Signalmolekül an sich oder zur Bindung von Signalmolekülen oder zur Stabilisierung. A ist bevorzugt am C-Terminus lokalisiert und ist beispielsweise ein Amin bevorzugt Lysin oder alternativ eine andere Aminosäure mit einem freien Amin wie z.B. Ornitin oder eine organischen Gruppe mit einem freien Amin an das ein Chelator zur Markierung mit Radionukliden oder ein MRT-Kontrastmittel, Fluoreszenzfarbstoff oder ein Chemotherapeutikum gekoppelt wird. Chelatoren sind beispielsweise DTPA (Diethylenetriaminepentaacetic acid, alternativ einsetzbar N,N-Bis(2-[bis(carboxymethyl)aminol-ethyl)glycine), alternativ DOTA (1,4,7,10-Tetraazacyclododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) und die bekannten Derivate aller genannten Chelatoren.

Die Hochzahl gibt an, an welcher Position innerhalb der Aminosäuresequenz sich die Attachementgruppe befindet.

GLP-1 (x-y)A$^{37}$

hierbei ist das GLP-1 Derivate umfasst, wobei x die Zahl 7 einnimmt, und wobei y die Zahl 36 ist. A ist eine am C-Terminus lokalisierte Attachementgruppe, zur Bindung von Signalmolekülen. Die Attachementgruppe ist das Amin Lysin.

Insbesondere sind bevorzugt die Peptidderivate:

1. MC 10: (DTPA-Lys$^{37}$) GLP-1 (7-36) amid

Die Synthese erfolgt z.B. über Firma Peptide Specialty Laboratories GmbH nach der Methode von Merrifield und Reinigung über HPLC.

MC 10 (DTPA-Lys$^{37}$) GLP-1 (7-36) amid besteht aus den Aminosäuren 7-36 von GLP-1 und trägt am C-terminalen Ende als Aminosäure mit einem freien Amin vorzugsweise Lysin an Position 37 sowie den Chelator DTPA.

Die erfindungsgemäßen Peptidderivate von GLP-1 die am C-Terminus über ein Amin modifiziert sind und über den N-Terminus an den GLP-1-Rezeptor binden, umfassen auch Moleküle, die sich von den Aminosäuresequenzen der beschriebenen Peptide GLP-1 an einer oder mehreren Positionen unterscheiden und einen hohen Grad an Homologie zu diesen Sequenzen aufweisen. Homologie meint dabei eine Sequenzidentität von mindestens 40%, insbesondere eine Identität von mindestens 60%, vorzugsweise über 80% und besonders bevorzugt über 90%. Die Abweichungen zu den oben beschriebenen Aminosäuresequenzen können dabei durch Deletion, Substitution und/oder Insertion entstanden sein.

## Markierung der Peptidderivate und der chimären Peptide

Die erfindungsgemäßen Peptidderivate werden zur Herstellung eines Mittels zur Diagnostik und Therapie gut- und bösartiger Erkrankungen, bei denen die GLP-1 Rezeptorexpression eine Rolle spielt, in einem geeigneten Stabilisierungspuffer gelöst, z.B. zur Stabilisierung der Metalle in vorzugsweise 0,5 M Natriumacetat pH 5,4 mit einer Konzentration von ca. $10^{-3}$ M. Alternativ wird zur Stabilisierung von Fluoreszenzfarbstoffen ein Puffer aus Ammoniumacetat bevorzugt, zur Stabilisierung von Chemotherapeutika und Kontrastmitteln ein physiologischer Puffer.

Die Markierung erfolgt an der Attachementgruppe A durch Koppelung von Radionukliden, MRT-Kontrastmitteln, Fluoreszenzfarbstoffen oder Chemotherapeutika. Dabei werden je nach Art der Anwendung für *in vivo* oder *in vitro* unterschiedliche Verfahren verwendet.

Als Radionuklide zur kovalenten oder komplexen Kopplung werden verwendet:

| Nuklid | Anwendungsverfahren | $t_{1/2}$[h] | Emittierende Strahlung | Energie[keV] | Art der Kopplung |
|---|---|---|---|---|---|
| F-18 | PET | 1,8 | $\beta^+$ | 634 | kovalent |
| Cu-64 | PET | 12,7 | $\beta^+$ | 1673 | komplex |
| Cu-67 | Therapie | 61,8 | $\beta^-$ <br> $\gamma$ | 391 <br> 184 | komplex |
| Ga-67 | SPECT | 79,2 | $\gamma$ | 93/184/300 | komplex |
| Ga-68 | PET | 1,1 | $\beta^+$ | 2921 | komplex |
| Y-86 | PET | 14,8 | $\beta^+$ <br> $\gamma$ | 1220 <br> 1076/1153 | komplex |
| Y-90 | Therapie | 64,1 | $\beta^-$ | 2280 | komplex |
| Tc-99m | SPECT | 6 | $\gamma$ | 140 | komplex |
| In-111 | SPECT | 67,2 | $\gamma$ | 171/245 | komplex |
| I-123 | SPECT | 13,2 | $\gamma$ | 158 | kovalent |

(fortgesetzt)

| Nuklid | Anwendungsverfahren | $t_{1/2}$[h] | Emittierende Strahlung | Energie[keV] | Art der Kopplung |
|---|---|---|---|---|---|
| I-124 | PET | 101 | $\beta^+$ $\gamma$ | 2137/1534 602 | kovalent |
| I-131 | Therapie | 192 | $\gamma$ $\beta^-$ | 364 606 | kovalent |
| Lu-177 | Therapie | 158 | $\gamma$ $\beta^-$ | 208 112/208 | komplex |
| Re-186 | Therapie | 88,8 | $\gamma$ $\beta^-$ | 137 1071 | komplex |
| Re-188 | Therapie | 17 | $\gamma$ $\beta^-$ | 155/477/632 1965/2120 | komplex |
| Pt-193m | Therapie | 104 | $\gamma$ Auger e$^-$ | 135 | komplex |
| Pt-195m | Therapie | 96 | $\gamma$ Auger e$^-$ | 98 | komplex |
| Ac-225 | Therapie | 240 | $\gamma$ $\alpha$ | 99, 150 | komplex |
| At-211 | Therapie | 7,2 | $\gamma$ Auger e$^-$ | 687 | komplex kovalent |
| Bi-213 | Therapie | 0,76 | $\gamma$ $\alpha$ | 440 | komplex |
| Sm-153 | Therapie | 46 | $\gamma$ $\beta^-$ | 103 | komplex |
| Er-169 | Therapie | 226 | $\beta^-$ | 100 | komplex |

PET (Positronen Emissions Tomographie), SPECT (Single Photonen Computer Tomographie)

Aus fluoreszierendem Farbstoff/Chromophor warden beispielsweise verwendet:

Fluorescein, Rhodamin, Coumarin, BODIPY, Pyrene (Cascadblau), Lucifergelb, Phycobiliprotein, Cyanin, Alexafluoro, Oregongrün, Texasrot und deren Derivate.

Chelatoren sind beispielsweise DTPA (Diethylenetriaminepentaacetic acid, N,N-Bis(2-[bis(carboxymethyl)amino]-ethyl)glycine), DOTA (1,4,7,10-Tetraazacyclodododecane-1,4,7,10-tetra-acetic acid), HYNIC (6-Hydrazinopyridin-3-carbonsäure), MAG3 (mercaptoacetyl-glycylglycylglycine), N4 (1,4,8,11-tetraazaundecane) oder die Derivate aller genannten Chelatoren.

MRT-Kontrastmittel sind zum Beispiel: Gadolinum, Mangan, Eisen, Europium, Kupfer, Nickel, Chrom, Prasodymium, Dysprosium oder Holmium oder deren Verbindung aber auch negative Kontrastmittel wie z.B. Perfluorocarbone sowie z.B. Isotope zur MRT Spektroskopie wie F-19, H-1, P-31, Na-19.

Negative Kontrastmittel sind erfindungsgemäß Kontrastmittel, die das MRT-Signal auslöschen bzw. stark abschwächen und nicht verstärken.

Chemotherapeutika sind insbesondere Alkylanzien, Interkalatoren, Antimetabolite, Enzymhemmer und Mitosegifte (beispielsweise Alkylsulfonate, Ethylimine Nitrosoharnstoffe Stickstofflostderivate, Folsäureanaloga, Purinanaloga, Pymiridinanaloga, Podophyllinderivate, Taxane, Vincaalkaloide, Anthrazykline, sonstige zytostatische Antibiotika, Platinverbindungen, Campthotecinderivate, verschiedene Hormone, Wachstumsfaktoren, Interferone oder Interleukine), ansonsten die in "Onkologie 2004/05" Autoren Preiss, Dornhoff, Hagmann, Schmieder erschienen im Zuckschwerdtverlag auf den Seiten 230-287 beschriebenen Chemotherapeutika, aber auch alle anderen zytostatisch oder zytotoxisch wirkenden Substanzen.

Je nach Art der Anwendung der erfindungsgemäßen Proteinderivate und dem aus ihnen hergestellten Mittel zur Diagnostik und Therapie gut- und bösartiger Erkrankungen, bei denen die GLP-1 Rezeptorexpression eine Rolle spielt, wird die Markierungsreaktion in zwei Varianten durchgeführt.

**Markierung zur *in vitro* Anwendung in der Radiotherapie**

3 μL der in einem geeigneten Stabilisierungspuffer vorzugsweise 0,5 M Natriumacetat pH 5,4 mit einer Konzentration von ca. $10^{-3}$ M gelösten erfindungsgemäßen Peptidderivate werden zur Markierung zu 500 μL 0,5M Natriumacetat pH 5,4 gegeben. Der pH-Wert liegt dabei zwischen 3-6. Dazu wird 185 MBq $^{111}InCl_3$ (Tyco, Petten, The Netherlands) in 0,1 M HCl 500 μL hinzugefügt und während 30 Minuten bei 370°C inkubiert. Um alle Bindungsstellen abzusättigen wird nochmals 3 μL $10^{-3}$ M Lösung $^{nat}InCl_3$ zugegeben und nochmals 30 min inkubiert.

Die Qualitätskontrolle wird über eine HPLC- Säule vorgenommen:

Säule: CC 250/4.6 Nucleosil 120-5 C18 (Machery-Nagel, Oenisingen, Schweiz) Gradient: 0->5 min 100% 0.05 M $NH_4OOCCH_3$, pH 5.4 (Puffer A); 5->25 min 100% Puffer A ->50% Puffer A/50% Acetonitril.

Die Qualitätskontrolle für eine in vitro Anwendung wird erfüllt bei einer Markierungsausbeute von mehr als 98%.

Somit steht ein radioaktiv markiertes Mittel zur Diagnostik und Therapie gut- und bösartiger Erkrankungen, bei denen die GLP-1 Rezeptorexpression eine Rolle spielt, zur Verfügung, dass beispielsweise in der Zell- und Gewebekultur mit Pankreaszellen eingesetzt wird.

**Markierung zur *in vivo* Anwendung in der Radiotherapie**

3 μL der in einem geeigneten Stabilisierungspuffer vorzugsweise 0,5 M Natriumacetat pH 5,4 mit einer Konzentration von ca. $10^{-3}$ M gelösten erfindungsgemäßen Peptidderivate werden zur Markierung zu 500 μL

0,5 M Natriumacetat pH 5,4 gegeben. Am Ende wird dazu 185 MBq $^{111}InCb$ (Tyco, Petten, The Netherlands) in 0,1 M HCl 500 μL dazugegeben und während 30 Minuten bei 370°C inkubiert. Die Qualitätskontrolle wird über eine HPLC-Säule vorgenommen:

Säule: CC 250/4.6 Nucleosil 120-5 C18 (Machery-Nagel, Oenisingen, Schweiz) Gradient: 0->5 min 100% 0.05 M $NH_4OOCCH_3$, pH 5.4 (Puffer A); 5->25 min 100% Puffer A ->50% Puffer A/50% Acetonitril.

Die Qualitätskontrolle für eine in vivo Anwendung wird erfüllt bei einer Markierungsausbeute von mehr als 98%.

Somit steht ein radioaktiv markiertes Mittel zur Diagnostik und Therapie gut- und bösartiger Erkrankungen, bei denen die GLP-1 Rezeptorexpression eine Rolle spielt zur Verfügung, dass beispielsweise zur Tumordetektion bei Patienten eingesetzt wird.

Der Begriff Patient bezieht sich dabei gleichermaßen auf Menschen und Wirbeltiere. Damit wird das Mittel in der Human- und Veterinärmedizin verwendet. Das therapeutisch und diagnostisch wirksame Mittel der vorliegenden Erfindung wird den Patienten, als Teil einer pharmazeutisch akzeptablen Komposition entweder oral, rektal, parenteral intravenös / intrararteriell, intramuskulär, subkutan, intrathekal, intracistemal, intracraniell, intravaginal, intraperitoneal, intravasculär, lokal (Puder, Salbe oder Tropfen) oder in Sprayform verabreicht. Die entsprechende Dosierung ist je nach Anwendungsfall für Diagnostik und Therapie gut- und bösartiger Erkrankungen, bei denen die GLP-1 Rezeptorexpression eine Rolle spielt, vom Arzt festzulegen.

**Internalisierungsstudie**

Die Intemalisierungsstudie zeigt beispielhaft den Transport der über in vitro-Markierung radioaktiv markierten erfindungsgemäßen Peptidderivate und chimären Proteine in die Zelle.

In einer 6-well Platte werden 100 000 GLP-1-Rezeptor transfizierte CHO Zellen ausgesät. Die Zellen wachsen bis sie konfluent sind. Es werden 4 Gruppen gebildet:

Gruppe 1: totale Bindung, PBS gewaschen

Es werden 100 000 cpm $^{111}In$ ($10^{-15}$ Mol) markiertes Peptid in 2 mL Medium zugegeben und 1 h bei 37°C inkubiert. Danach wird 3x mit PBS gewaschen und die Zellen mit 20 mM MOPS (3-Morpholinopropanesulfonic-acid) + 0,1% Triton-X-100 (pH 7,4) abgelöst. Die Aufnahme (Uptake) in die Zellen wird im γ-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/μg Protein angegeben.

Gruppe 2: unspezifische Bindung, PBS gewaschen

Es werden 20 $\mu$L einer $10^{-3}$ M GLP-1 Lösung und 100 000 cpm $^{111}$In markiertes Peptid in 2 mL Medium zugegeben und 1 h bei 37°C inkubiert. Danach wird 3x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im $\gamma$-Counter gemessen. Die Zellzahl wird über der Proteingehalt mit dem Proteinassay-Kit von Bio-Rad (München, Deutschland) basierend auf der Methode von Bradford gemessen. Das Resultat wird in cpm/$\mu$g Protein angegeben.

Gruppe 3: totale Bindung, Säure gewaschen

Es werden 20 $\mu$L einer $10^{-3}$ M GLP-1 Lösung und 100 000 cpm $^{111}$In markiertes Peptid in 2 mL Medium zugegeben und 1 h bei 37°C inkubiert. Danach wird 1 x mit 0,1 M Natrium Acetatpuffer pH 4 gewaschen und 2x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im $\gamma$-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/$\mu$g Protein angegeben.

Gruppe 4: unspezifische Bindung, Säure gewaschen

Es werden 20 $\mu$L einer $10^{-4}$ M GLP-1 Lösung und 100 000 cpm $^{111}$In markiertes Peptid in 2 mL Medium zugegeben und 1 h bei 37°C inkubiert. Danach wird 1 x mit 0,1 M Natrium Acetatpuffer pH 4 gewaschen und 2x mit PBS gewaschen und die Zellen mit 20 mM MOPS (3-Morpholinopropansulfonylsäure) + 0,1% Triton-X-100 (pH 7,4) abgelöst. Der Uptake in den Zellen wird im $\gamma$-Counter gemessen. Die Zellzahl wird über den Proteingehalt mit dem Proteinassay-Kit von Bio-Rad (München, Deutschland) basierend auf der Methode von Bradford gemessen. Das Resultat wird in cpm/$\mu$g Protein angegeben.

$$\text{Auswertung: } \%IDsB = \frac{\text{Res3.} - \text{Res4.}}{\text{Res1.} - \text{Res2.}} *100$$

%IdsB = %Internalisierung der spezifischen Bindung

|      | %IdsB    |
|------|----------|
| MC10 | $75\pm5$ |
| MC11 | $70\pm7$ |
| MC12 | $73\pm9$ |

Das Ergebnis zeigt, dass ein guter Transport in die Zellen erfolgt.

**Bindungsstudien**

Bindungsstudien zeigen die spezifische Bindung der über in vivo-Markierung radioaktiv markierten erfindungsgemäßen Peptidderivate und Chimären Proteine an den GLP-1 -Rezeptor.
In einer 6-well Platte werden 100 000 GLP-1-Rezeptor transfizierte CHO Zellen ausgesät. Die Zellen wachsen bis sie konfluent sind. Es werden dann in 2 mL 100 000 cpm $^{111}$In markiertes Peptid zugegeben. Um die Bindung zu testen wird mit 20 $\mu$L einer $10^{-3}$ M GLP-1 Lösung geblockt.

|      | %Blockierung |
|------|--------------|
| MC10 | $80\pm3$     |

Die *in vivo* Bioverteilung wird beispielsweise mit Nagern z.B. mit Nacktmäusen dargestellt. Dazu werden GLP-1 transfizierte CHO Zellen in Nacktmäuse injiziert. Nach ca. 3-5 Wochen sind Tumore einer Größe von ca. 300 mg gewachsen. Den Mäusen wird eines der erfindungsgemäßen 37 MBq $^{111}$In markierten Peptide über die Schwanzvene injiziert und die Mäuse werden unter der einer γ-Kamera nach 4 h gemessen.

Dabei erfolgt eine schnelle Clearance über die Niere und eine Aufnahme in der Niere. Eine hoher Aufnahme erfolgt ebenfalls im GLP-1 Rezeptor positivem Tumor, während ein GLP-1 Rezeptor negativer Tumor kaum eine Aufnahme zeigt. Eine leichte Aufnahme ist ebenfalls im Pankreas zu sehen, andere Organe zeigen keine sichtbare Aufnahme.

In Gruppen von jeweils 4 Mäusen werden *ex vivo* Bioverteilungsstudien durchgeführt, bei denen 555 kBq In-111 markiertes MC10 in die Schwanzvene injiziert werden. 1,4 und 24 h p.i. werden die Mäuse getötet und die Organe entnommen.

Die Aufnahme der Radioaktivität wird gemessen und die Organe gewogen. Die % injizierte Dosis pro Gramm Organgewicht (% i.D./g) wird berechnet. Die Resultate sehen wie folgt aus:

| Organ | 1 h | Staabw | 4 h | Staabw | 24 h | Staabw |
|---|---|---|---|---|---|---|
| Blut | 0,01 | 0,00 | 0,00 | 0,00 | 0,00 | 0,00 |
| Leber | 0,03 | 0,01 | 0,01 | 0,01 | 0,01 | 0,00 |
| Magen | 0,14 | 0,10 | 0,13 | 0,07 | 0,07 | 0,06 |
| Milz | 0,02 | 0,02 | 0,01 | 0,00 | 0,01 | 0,00 |
| Pankreas | 0,58 | 0,50 | 0,62 | 0,28 | 0,37 | 0,27 |
| Nieren | 7,90 | 3,62 | 7,41 | 3,56 | 4,85 | 3,05 |
| Darm | 0,12 | 0,06 | 0,07 | 0,06 | 0,04 | 0,03 |
| Lunge | 0,80 | 0,56 | 0,36 | 0,17 | 0,22 | 0,07 |
| Herz | 0,02 | 0,01 | 0,01 | 0,01 | 0,00 | 0,00 |
| Knochen | 0,02 | 0,04 | 0,01 | 0,00 | 0,01 | 0,01 |
| Muskel | 0,01 | 0,00 | 0,00 | 0,00 | 0,00 | 0,00 |
| Tumor - | 0,03 | 0,03 | 0,02 | 0,01 | 0,01 | 0,00 |
| Tumor + | 0,42 | 0,19 | 0,31 | 0,30 | 0,20 | 0,16 |

Mittelwert aus der Bioverteilung mit 4 Mäusen pro Gruppe in % i.D./g Staabw: Standardabweichung.

**Patentansprüche**

1. Peptidderivate von GLP-1, welche ganz oder teilweise die Aminosäuresequenz

(H)-$_1$His-$_2$Asp-$_3$Glu-$_4$Phe-$_5$Glu-$_6$Arg-$_7$His-$_8$Ala-$_9$Glu-$_{10}$Gly-$_{11}$Thr-$_{12}$Phe-$_{13}$Thr-$_{14}$Ser-$_{15}$Asp-$_{16}$Val-$_{17}$Ser-$_{18}$Ser-$_{19}$Tyr-$_{20}$Leu-$_{21}$Glu-$_{22}$Gly-$_{23}$Gln-$_{24}$Ala-$_{25}$Ala-$_{26}$Lys-$_{27}$Glu-$_{28}$Phe-$_{29}$Ile-$_{30}$Ala-$_{31}$Trp-$_{32}$Leu-$_{33}$Val-$_{34}$Lys-$_{35}$Gly-$_{36}$Arg-$_{37}$Gly-(OH)

des Peptides GLP-1(1-37) beinhalten,
**dadurch gekennzeichnet,**

■ **dass** sie am C-Terminus über ein Amin modifiziert sind und über den N-Terminus an den GLP-1-Rezeptor binden und
■ dass sie aufgebaut sind aus GLP-1(x-y)A wobei x die Zahl7 annimmt, und wobei y die Zahl36 ist, und wobei A eine am C-Terminus lokalisierte Attachementgruppe zur Bindung von Signalmolekülen ist
■ und dass die Attachementgruppe A Lysin ist.

2. Peptidderivate von GLP-1 gemäß Anspruch 1 **dadurch gekennzeichnet, dass** die Attachementgruppe A eine Aminosäure mit einem freien Amin wie Ornithin ist oder eine organische Gruppe mit einem freien Amin.

3. Peptidderivate von GLP1 gemäß einem der Ansprüche 1 bis 2, **dadurch gekennzeichnet, dass** an die Attachementgruppe A ein Chelator zur Markierung mit Radionukliden oder ein MRT-Kontrastmittel, Fluoreszenzfarbstoff oder ein Chemotherapeutikum gekoppelt wird.

4. Peptidderivate von GLP-1 gemäß der vorangegangenen Ansprüche, **dadurch gekennzeichnet, dass** der Chelator N,N-Bis(2-[bis(carboxymethyl)amino)-ethyl]glycine), DOTA (1,4,7,10-Tetraazacyclododecane-1,4,7,10-tetra-acetic acid), HYNIC (6-Hydrazinopyridin-3-carbonsäure), MAG3 (mercaptoacetylglycylglycylglycine), N4 (1,4,8,11-tetraazaundecane) und deren bekannte Derivate ist, vorzugsweise DTPA (Diethylenetriamine-pentaacetic acid).

5. Peptidderivate von GLP-1 gemäß der vorangegangenen Ansprüche, **dadurch gekennzeichnet, dass** die Markierung eine Koppelung von Radionuklid, MRT-Kontrastmittel, Fluoreszenzfarbstoff und/oder Chemotherapeutikum ist.

6. Peptidderivate von GLP-1 gemäß der vorangegangenen Ansprüche, **dadurch gekennzeichnet, dass** Fluoreszenzfarbstoffe ausgewählt sind insbesondere aus der Gruppe Fluorescein, Rhodamin, Coumarin, BODIPY, Pyrene (Cascadblau), Lucifergelb, Phycobiliprotein, Cyanin, Alexafluor, Oregongrün, Texasrot, Coumarin und deren Derivaten.

7. Peptidderivate von GLP-1 gemäß der vorangegangenen Ansprüche, **dadurch gekennzeichnet, dass** das Radionuklid ausgewählt ist insbesondere aus der Gruppe F-18, Cu-64, Ga-67, Ga-68, Y-86, Y-90, Tc-99m, In-111, I-123, I-124,-I-131, Lu-177, Re-186, Re-188, Pt-193m, Pt-195m, Ac-225, At-211, Bi-213, Sm-153 oder Er-169.

8. Peptidderivate von GLP-1 gemäß der vorangegangenen Ansprüche, **dadurch gekennzeichnet, dass** MRT Kontrastmittel Gadolinium, Mangan, Eisen, Europium, Kupfer, Nickel, Chrom, Prasodymium, Dysprosium oder Holmium oder deren Verbindung ist oder Perfluorocarbone oder F-19, H1-, P31, Na-19 ist.

9. Peptidderivate von GLP-1 gemäß der vorangegangenen Ansprüche, **dadurch gekennzeichnet, dass** das Chemotherapeutikum ausgewählt ist insbesondere aus der Gruppe Alkylsulfonate, Ethylimine, Nitrosoharstoffe, Stickstofflostderivate, Folsäureanaloga, Purinanaloga, Pyrimidinanaloga, Podophyllinderivate, Taxane, Vincaalkaloide, Anthrazykline, sonstige zytostatische Antibiotika, Platinverbindungen, Campthotecinderivate, Hormone, Wachstumsfaktoren, Interferone oder Interleukine oder zytostatisch oder zytotoxisch wirkende Substanzen.

10. Peptidderivate von GLP-1 gemäß der vorangegangenen Ansprüche **dadurch gekennzeichnet, dass** die Markierung durch Koppelung von Radionukliden für in-vitro-Anwendungen durch Absättigung der Bindungsstellen mit $^{nat}InCl_3$ erfolgt.

11. Verwendung der Peptidderivate von GLP-1 gemäß der vorangegangenen Ansprüche zur Herstellung eines Mittels zur Diagnostik und Therapie gut- und bösartiger Erkrankungen, bei denen die GLP-1 Rezeptorexpression eine Rolle spielt.

12. Peptidderivate von GLP-1 gemäß der Ansprüche 1 bis 10 zur Verwendung bei der Bestimmung der Dichte Insulin-produzierender Zellen in einem Gewebe.

13. Peptidderivate von GLP-1 gemäß der Ansprüche 1 bis 10 zur Verwendung bei der Bestimmung der Expression von GLP-1 -Rezeptoren oder deren Dichte.

14. Mittel zur Diagnostik und Therapie gut- und bösartiger Erkrankungen, bei denen die GLP-1 Rezeptorexpression eine Rolle spielt, **dadurch gekennzeichnet dass** es markierte Peptidderivate von GLP-1 gemäß Anspruch 1 enthält.

15. Mittel zur Diagnostik und Therapie gut- und bösartiger Erkrankungen, bei denen die GLP-1 Rezeptorexpression eine Rolle spielt, **dadurch gekennzeichnet, dass** es unmarkierte Peptidderivate von GLP-1 gemäß Anspruch 1 enthält.

16. Mittel gemäß Anspruch 14 **dadurch gekennzeichnet dass** die Markierung eine Koppelung von Radionukliden, MRT Kontrastmitteln, Fluoreszenzfarbstoffen und/oder Chemotherapeutikum beinhaltet.

17. Mittel gemäß Anspruch 14 und Anspruch 15 zur Verwendung bei der Diagnostik und Therapie gut- und bösartiger Erkrankungen, bei denen die GLP-1 Rezeptorexpression eine Rolle spielt.

18. Mittel gemäß Anspruch 14 und Anspruch 15 zur Verwendung in der Diagnostik und Therapie, neuroendokriner Tumoren (NET) insbesondere Insulinomen und kleinzelligen Bronchialkarzinomen.

19. Mittel gemäß Anspruch 14 zur Verwendung in der Szintigraphie, PET, SPECT, MRT, optischen Diagnostik, rezeptorvermittelten Chemotherapie, rezeptorvermittelten, zytostatischen oder zytotoxischen Therapie und Radiopeptidtherapie.

**Claims**

1.  GLP-1 peptide derivates containing in whole or in part the amino acid sequences

    $(H)\text{-}_1His\text{-}_2Asp\text{-}_3Glu\text{-}_4Phe\_5Glu\text{-}_6Arg\text{-}_7His\text{-}_8Ala\text{-}_9Glu\text{-}_{10}Gly\text{-}_{11}Thr\text{-}_{12}Phe\text{-}_{13}Thr\text{-}_{14}Ser\text{-}_{15}Asp\text{-}_{16}Val\text{-}_{17}Ser\text{-}_{18}Ser\text{-}_{19}Tyr\text{-}_{20}Leu\text{-}_{21}Glu\text{-}_{22}Gly\text{-}_{23}Gln\text{-}_{24}Ala\text{-}_{25}Ala\text{-}_{26}Lys\text{-}_{27}Glu\text{-}_{28}Phe\text{-}_{29}Ile\text{-}_{30}Ala\text{-}_{31}Trp\text{-}_{32}Leu\text{-}_{33}Val\text{-}_{34}Lys\text{-}_{35}Gly\text{-}_{36}Arg\text{-}_{37}Gly\text{-}_{(OH)}$

    the peptide GLP-1 (1-37),
    **characterized in that**

    ■ they are modified at the C-terminus by an amine and bind to the GLP-1 receptor by the N-terminus, and
    ■ that they are composed of GLP-1 (x-y)A with x being the number 7 and y being the number 36, and with A being an attachment group localized at the C-terminus to bind signal molecules,
    ■ and that the attachment group A is lysine.

2.  GLP-1 peptide derivates according to Claim 1, **characterized in that** the attachment group A is an amino acid with a free amine such as ornithine, or an organic group with a free amine.

3.  GLP-1 peptide derivates according to one of the Claims 1 to 2, **characterized in that** to the attachment group A is being coupled a chelating agent for marking with radionuclides, or an MRT-contrast medium, fluorescent dye, or a chemotherapeutant.

4.  GLP-1 peptide derivatives according to the preceding Claims, **characterized in that** the chelating agent is N,N-Bis (2[bis(carboxymethyl)amino]-ethyl)glycine), DOTA (1,4,7,10-tetraazacyclododecane-1,4,7,10-tetra-acetic acid, HYNIC (6-hydrazinopyridine-3-carboxylic acid), MAG3 (mercaptoacetylglycylglycylglycine), N4 (1,4,8,11-tetraazaundecane) and their known derivates, preferably DTPA (diethylenetriamine-pentaacetic acid).

5.  GLP-1 peptide derivates according to the preceding Claims, **characterized in that** the marking is a coupling of radionuclide, MRT-contrast medium, fluorescent dye, and/or chemotherapeutant.

6.  GLP-1 peptide derivates according to the preceding Claims, **characterized in that** fluorescent dyes are chosen especially from the group of fluorescein, rhodamine, coumarin, BODIPY, pyrene (cascade blue), Lucifer yellow, phycobili protein, cyanine, alexa fluor, Oregon green, Texas red, coumarin, and their derivates.

7.  GLP-1 peptide derivates according to the preceding Claims, **characterized in that** the radionuclide is chosen especially from the group of F-18, Cu-64, Ga-67, Ga-68, Y-86, Y-90, Tc-99m, I n-111, I-123, I-124, I-131, Lu-177, Re-186, Re-188, Pt-195m, Ac-225, At-211, Bi-213, Sm-153, or Er-169.

8.  GLP-1 peptide derivates according to the preceding Claims, **characterized in that** the MRT contrast medium is gadolinium, manganese, iron, europium, copper, nickel, chrome, praseodymium, dysprosium, or holmium, or their compound, or perfluorocarbon, or F-19, H1-, P31, Na-1 9.

9.  GLP-1 peptide derivates according to the preceding Claims, **characterized in that** the chemotherapeutant is chosen especially from the group of alkyle sulphonates, ethylimines, nitrosohar agents, nitrogen mustard derivates, folic acid analogues, purine analogues, pyrimidine analogues, podophylline derivates, taxanes, Vinca alkaloids, anthracyclines, other cytostatic antibiotics, platinum compounds, campthotecine derivates, hormones, growth factors, interferons or interleukins, or substances of cytostatic or cytotoxic effect.

10. GLP-1 peptide derivates according to the preceding Claims, **characterized in that** the marking is done by coupling radionuclides for in-vitro applications by saturation of the bonding points with $^{nat}InCl_3$.

11. Use of the GLP-1 peptide derivates according to the preceding Claims to prepare an agent for the diagnosis and therapy of benign and malign diseases in which the GLP-1 receptor expression is a factor.

12. GLP-1 peptide derivates according to the Claims 1 to 10 for use with the determination of the density of insulin-producing cells in a tissue.

13. GLP-1 peptide derivates according to the Claims 1 to 10 for use with the determination of the expression of GLP-1 receptors or their density.

14. Agent for the diagnosis and therapy of benign and malign diseases in which the GLP-1 receptor expression is a factor, **characterized in that** it contains marked GLP-1 peptide derivates according to Claim 1.

15. Agent for the diagnosis and therapy of benign and malign diseases in which the GLP-1 receptor expression is a factor, **characterized in that** it contains unmarked GLP-1 peptide derivates according to Claim 1.

16. Agent according to Claim 14, **characterized in that** the marking contains a coupling of radionuclides, MRT contrast mediums, fluorescent dyes, and/or chemotherapeutant.

17. Agent according to Claim 14 and Claim 15 for use with the diagnosis and therapy of benign and malign diseases in which the GLP-1 receptor expression is a factor.

18. Agent according to Claim 14 and Claim 15 for use in the diagnosis and therapy of neuroendocrine tumors (NET), especially of insulinomae and oat cell carcinoma.

19. Agent according to Claim 14 for use in scintigraphy, PET, SPECT, MRT, optical diagnosis, receptor-indicated chemotherapy, receptor-indicated, cytostatic or cytotoxic therapy, and in radiopeptide therapy

**Revendications**

1. Dérivés de peptide GLP-1 contenant entièrement ou partiellement la séquence aminoacide

(H)-$_1$His-$_2$Asp-$_3$Glu-$_4$Phe-$_5$Glu-$_6$Arg-$_7$His-$_8$Ala-$_9$Glu-$_{10}$Gly-$_{11}$Thr-$_{12}$Phe-$_{13}$Thr-$_{14}$Ser-$_{15}$Asp-$_{16}$Val-$_{17}$Ser-$_{18}$Ser-$_{19}$Tyr-$_{20}$Leu-$_{21}$Glu-$_{22}$Gly-$_{23}$Gly-$_{24}$Ala-$_{25}$Ala-$_{26}$Lys-$_{27}$Glu-$_{28}$Phe-$_{29}$Ile-$_{30}$Ala-$_{31}$Trp-$_{32}$Leu-$_{33}$Val-$_{34}$Lys-$_{35}$Gly-$_{36}$Arg-$_{37}$Gly-$_{(OH)}$

du peptide GLP-1 (1-37),
**caractérisés en ce**

■ **qu'**au C-terminal ils sont modifiés par une amine et qu'ils fixent par le N-terminal sur le récepteur GLP-1, et
■ qu'ils sont composés de GLP-1 (x-y)A avec x étant l'indice 7 et y l'indice 36, et avec A étant un groupe d'attachement localisé au C-terminal pour fixer des molécules-signaux,
■ et que le groupe d'attachement A est lysine.

2. Dérivés de peptide GLP-1 selon la Revendication 1, **caractérisés en ce que** le groupe d'attachement A est un aminoacide avec une amine libre comme ornithine, ou un groupe organique avec une amine libre.

3. Dérivés de peptide GLP-1 selon une des Revendications 1 à 2, **caractérisés en ce qu'** au groupe d'attachement A est couplé un agent chélateur pour marquage avec des radionucléides, ou un moyen de contraste, un colorant fluorescent, ou une chimiothérapeutique.

4. Dérivés de peptide GLP-1 selon les Revendications précédentes, **caractérisés en ce que** l'agent chélateur est N, N-Bis(2-[bis(carboxymethyl)amino]-ethyl)glycine), DOTA (1,4,7,10-tétraazacyclododécane-1,4,7,10-acide tétra-acétique), HYNIC (6-hydrazinopyridine-3-acide carboxylique), MAG3 (mercaptoacétylglycilglycilglycine), N4 (1,4,8,11-tétraazaundécane) et leurs dérivés connus, de préférence DTPA (diéthylenetriamine-acide penta-acétique).

5. Dérivés de peptide GLP-1 selon les Revendications précédentes, **caractérisés en ce que** le marquage est un couplage de radionucléide, moyen de contraste MRT, colorant fluorescent, et/ou chimiothérapeutique.

6. Dérivés de peptide GLP-1 selon une des Revendications précédentes, **caractérisé en ce que** des colorants fluorescents sont choisis particulièrement de la fonction fluorescéine, rhodamine, coumarine, BODIPY, pyrène (bleu cascade), jaune Lucifer, phycobili-protéine, cyanine, fluor alexa, vert Oregon, rouge Texas, coumarine, et leurs dérivés.

7. Dérivés de peptide GLP-1 selon les Revendications précédentes, **caractérisés en ce que** le radionucléide est choisi particulièrement de la fonction F-18, Cu-64, Ga-67, Ga-68, Y-86, Y-90, Tc-99m, In-111, I-123, I-124, I-131, Lu-177, Re-186, Re-188, Pt-195m, Ac-225, At-211, Bi-213, Sm-153, ou Er-169.

8. Dérivés de peptide GLP-1 selon les Revendications précédentes, **caractérises en ce que** le moyen de contraste MRT est gadolinium, manganèse, fer, europium, cuivre, nickel, chrome, prasodymium, dysprosium, ou holmium, ou leurs composés, ou carbone perfluorique, ou F-19, H1, P31, Na-19.

9. Dérivés de peptide GLP-1 selon les Revendications précédentes, **caractérisés en ce que** la chimiothérapeutique est choisie particulièrement de la fonction sulfonates d'alkyle, ethylimines, agents nitrosohars, dérivés de lost, analogues d'acide folique, analogues de purine, analogues de pyrimidine, dérivés de podophylline, taxanes, alcaloïdes de Vinca, anthracyclines, autres antibiotiques cytostatiques, composés de platine, dérivés de campthotecine, hormones, facteurs de croissance, interferons ou interleukines, ou substances d'effet cytostatique ou cytotoxique.

10. Dérivés de peptide GLP-1 selon les Revendications précédentes, **caractérisés en ce que** le marquage est fait par couplage de radionucléides pour des applications in vitro par la saturation des points de fixation avec $^{nat}InCl_3$.

11. Emploi des dérivés de peptide GLP-1 selon les Revendication précédentes pour la production d'un agent pour la diagnose et thérapie des maladies bénignes et malignes, auxquelles l'expression du récepteur GLP-1 joue un rôle.

12. Dérivés de peptide GLP-1 selon les Revendications 1 à 10 pour l'emploi dans la détermination de la densité des cellules productrices d'insuline dans un tissu.

13. Dérivés de peptide GLP-1 selon les Revendications 1 à 10 pour l'emploi dans la détermination de l'expression des récepteurs GLP-1 ou leur densité.

14. Agent pour la diagnose et la thérapie des maladies bénignes et malignes auxquelles l'expression du récepteur GLP-1 joue un rôle, **caractérisé en ce que** qu'il contient des dérivés de peptide GLP-1 marqués.

15. Agent pour la diagnose et la thérapie des maladies bénignes et malignes auxquelles l'expression du récepteur GLP-1 joue un rôle, **caractérisé en ce que** qu'il contient des dérivés de peptide GLP-1 non-marqués.

16. Agent selon la Revendication 14, **caractérisée en ce que** le marquage contient un couplage de radionucléides, moyens de contraste MRT, colorants fluorescents, et/ou une chimiothérapeutique.

17. Agent selon la revendication 14 et la revendication 15 pour l'emploi dans la diagnostique et la thérapie des maladies bénignes et malignes, auxquelles l'expression du récepteur GLP-1 joue un rôle.

18. Agent selon la Revendication 14 et la revendication 15 pour l'emploi dans la diagnostique et la thérapie des tumeurs neuroendocrines (NET), particulièrement des insulinomae et des carcinomes bronchiques microcellulaires.

19. Agent selon la Revendication 14 pour l'emploi dans la scintigraphie, PET, SPECT, MRT, diagnostique optique, chimiothérapie indiquée par récepteur, la thérapie indiquée par récepteur, cytostatique ou cytotoxique, et dans la thérapie radiopeptide.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69018226 T2

- US 20030232761 A1

**In der Beschreibung aufgeführte Nicht-Patentliteratur**

- **Autoren Preiss ; Dornhoff, Hagmann.** Onkologie 2004/05. *Schmieder erschienen im Zuckschwerdtverlag,* 230-287